(19) Europäisches Patentamt
European Patent Office
Office européen des brevets

(11) Veröffentlichungsnummer: **0 175 151 B1**

# (12) EUROPÄISCHE PATENTSCHRIFT

(45) Veröffentlichungstag der Patentschrift: **18.01.89**

(51) Int. Cl.⁴: **C 07 C 79/32**, C 07 C 76/02, G 03 C 7/32 // C07C91/44

(21) Anmeldenummer: **85110315.0**

(22) Anmeldetag: **17.08.85**

(54) **2,4-Dichlor-3-alkyl-6-nitrophenole sowie ein Verfahren zu deren Herstellung.**

(30) Priorität: **29.08.84 DE 3431687**

(43) Veröffentlichungstag der Anmeldung: **26.03.86 Patentblatt 86/13**

(45) Bekanntmachung des Hinweises auf die Patenterteilung: **18.01.89 Patentblatt 89/3**

(84) Benannte Vertragsstaaten: **BE CH DE FR GB LI**

(56) Entgegenhaltungen:
**DE-A- 2 216 804**
**DE-A- 2 614 264**
**FR-A- 2 297 836**
**US-A- 3 772 002**

**CHEMICAL ABSTRACTS, Band 95, Nr. 9, 31. August 1981, Seite 728, Nr. 80326k, Columbus, Ohio, US; L.M. WEINSTOCK et al.: "Isomer-selective syntheses of 2,6-dichloro-4-nitrotoluene and 2,4-difluoroaniline" & ORG. PREP. PROCED. INT. 1981, 13(2), 103-8**
**CHEMICAL ABSTRACTS, Band 82, Nr. 13, 31. März 1975, Seite 504, Nr. 86163h, Columbus, Ohio, US; P. BUCHWALD et al.: "Synthesis of**

(73) Patentinhaber: **BAYER AG, Konzernverwaltung RP Patentabteilung, D-5090 Leverkusen 1 Bayerwerk (DE)**

(72) Erfinder: **Paetz, Christian, Dr., Hülsenanger 6, D-5060 Bergisch Gladbach 2 (DE)**
Erfinder: **Wedemeyer, Karlfried, Dr., Bilharzstrasse 7, D-5000 Köln 80 (DE)**

(56) Entgegenhaltungen: (Fortsetzung)
**2,6-dichlorobenzaldoxime from 4-nitro-toluene" & REV. ROUM. CHIM. 1974, 19(7), 1221-5**
**Ullmann, Band 9 (1975), S. 504-506 und 510-514**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 175 151 B1

ACTORUM AG

**Beschreibung**

Die Erfindung betrifft ein Verfahren zur Herstellung der neuen 2,4-Dichlor-3-alkyl-6-nitrophenole.

Es ist bekannt, 2,4-Dichlor-3-methyl-6-nitrophenol herzustellen, indem man ausgehend von 4-Chlor-5-methylphenol dieses zunächst sulfoniert, dann chloriert und anschliessend nitriert (vgl. z.B. DE-AS 2 501 899, DE-OS 2 216 804, GB-PS 1 361 714 und JP 7 234 326).

Nachteilig bei diesem Verfahren sind zum einen die Vielzahl der Reaktionsstufen, die zu durchlaufen sind, zum anderen die Schwerzugänglichkeit der benötigten Ausgangsprodukte. m-Alkylphenole, z.B. m-Kresol, und die daraus hergestellten 4-Chlor-5-alkylphenole lassen sich bekanntermassen nur recht schwer rein darstellen. So wird beispielsweise für die Herstellung von m-Ethylphenol in der DE-OS 2 229 776 die Diazotierung und Verkochung von m-Aminoacetophenon beschrieben, das durch Nitrierung von Acetophenon und anschliessender Reduktion hergestellt wurde. In der Europäischen Patentschrift 80 880 wird ein Verfahren zur Herstellung von m-Alkylphenolen beschrieben, bei dem man zunächst Alkylbenzole zu einem o-, m-, p-Isomerengemisch sulfoniert, anschliessend die gebildeten o- und p-Alkylbenzolsulfonsäuren desulfoniert und nachfolgend die verbleibende m-Alkylbenzolsulfonsäure einer Alkalischmelze unterwirft. Eine weitere Möglichkeit zur Herstellung von m-Alkylphenol besteht in der Isomerisierung von relativ leicht zugänglichem o-Alkylphenol an einem Kontakt (Houben-Weyl, Methoden der organischen Chemie, Bd. VI/1c, S. 1073–1081). Dabei entsteht ein o-, m-, p-Isomerengemisch an Alkylphenolen. Der Nachteil dieses Verfahrens besteht darin, dass neben der Isomerisierung auch Transalkylierung auftritt, d.h. die Bildung von Phenol und höher alkylierten Phenolen, und dieses Verfahren nur bei Alkylgruppen angewandt werden kann, die selbst stabile Carbokationen darstellen. Weiterhin ist von Nachteil, dass bei der erforderlichen destillativen Aufarbeitung des Isomerengemisches der m-Alkylphenolanteil aufgrund zu geringer Siedepunktsunterschiede nur als m-/p-Isomerengemisch abgetrennt werden kann. Um aus dem m-/p-Isomerengemisch das m-Alkylphenol abzutrennen, ist eine selektive Chlorierung mit relativ teurem Sulfurylchlorid erforderlich, wobei das m-Alkylphenol zu 4-Chlor-5-alkylphenol chloriert wird, das nunmehr einer destillativen Trennung leicht zugänglich ist. Nachteilig bei diesem Trennverfahren ist jedoch, dass neben dem gewünschten 4-Chlor-5-alkylphenol noch die entsprechenden o-Chlorphenole auftreten, die bis zu 40 Gew.-% der Ausbeute ausmachen können.

Es wurde nun ein Verfahren zur Herstellung von 2,4-Dichlor-3-alkyl-6-nitrophenolen der Formel (I)

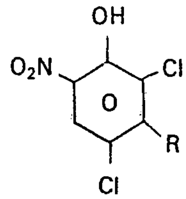

(I),

in der

R für einen Alkylrest mit 2 bis 8 Kohlenstoffatomen steht, gefunden, das dadurch gekennzeichnet ist, dass man 1-Alkyl-4-nitrobenzole der Formel (II)

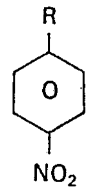

(II),

in der

R die obengenannte Bedeutung hat, in Gegenwart eines Katalysators mit Chlor bis zu einem Gehalt an Trichloralkylnitrobenzol von mindestens 60 Gew.-% im Chlorierungsgemisch chloriert, das Chlorierungsgemisch vom Katalysator befreit, anschliessend das Chlorierungsgemisch mit einem Hydrolysegemisch, das aus Wasser und aus einem mit Wasser mischbaren organischen Lösungsmittel sowie einer alkalisch reagierenden Verbindung besteht, hydrolysiert, das ausfallende Reaktionsprodukt abtrennt und mit einer wässrigen anorganischen Säure behandelt.

Die nach den erfindungsgemässen Verfahren hergestellten 2,4-Dichlor-3-alkyl-6-nitrophenole der allgemeinen Formel (I)

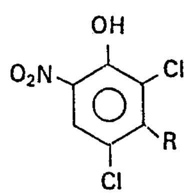

(I),

in der

R für einen Alkylrest mit 2 bis 8 Kohlenstoffatomen steht, sind neu.

In das erfindungsgemässe Verfahren werden bevorzugt als 1-Alkyl-4-nitrobenzole solche mit einem $C_2$–$C_6$–, besonders bevorzugt solche mit einem $C_2$–$C_4$-Alkylrest eingesetzt.

Als Alkylreste seien beispielsweise genannt der Ethyl-, der Propyl-, der Butyl-, der Pentyl-, der Hexyl-, der Cyclohexyl-, der Heptyl- und der Octylrest, bevorzugt der Ethyl-, der Propyl- und der Butylrest.

Als neue 2,4-Dichlor-3-alkyl-6-nitrophenole, die nach dem erfindungsgemässen Verfahren hergestellt werden können, seien genannt: 2,4-Dichlor-3-ethyl-6-nitrophenol, 2,4-Dichlor-3-isopropyl-6-nitrophenol, 2,4-Dichlor-3-tert.-butyl-6-nitrophenol, 2,4-Dichlor-3-cyclohexyl- 6-nitrophenol und 2,4-Dichlor-3-isoamyl- 6-nitrophenol, bevorzugt 2,4-Dichlor-3-ethyl- 6-nitrophenol, 2,4-Dichlor-3-isopropyl- 6-nitrophenol und 2,4-Dichlor-3-tert.-butyl- 6-nitrophenol.

Als Katalysatoren können in das erfindungsgemässe Verfahren alle bekannten Chlorierungskatalysatoren eingesetzt werden, wie sie beispielsweise in Houben.Weyl, Bd. V/3, Seite 651–725 beschrieben sind. Beispielsweise seien genannt:

Eisen(III)chlorid, Antimon(III)chlorid, Aluminium(III)chlorid und Jod. Die Chlorierungskatalysatoren können in das erfindungsgemässe Verfahren allein oder im Gemisch untereinander eingesetzt werden.

Die Menge des Chlorierungskatalysators ist für das erfindungsgemässe Verfahren nicht kritisch und kann leicht durch Vorversuche ermittelt werden. Üblicherweise setzt man den Katalysator in einer Menge von etwa 1 bis 10, bevorzugt 4 bis 7 Gew.-%, bezogen auf das eingesetzte 1-Alkyl-4-nitrobenzol, in das erfindungsgemässe Verfahren ein.

Zur Chlorierung der 1-Alkyl-4-nitrobenzole kann man diese sowohl in Substanz als auch gelöst in einem inerten, organischen Lösungsmittel einsetzen. Als inerte, organische Lösungsmittel kommen bevorzugt halogenierte aliphatische Kohlenwasserstoffe in Frage, wie Methylenchlorid, Chloroform und/oder Tetrachlorethan. Es ist jedoch auch möglich, andere inerte, organische Lösungsmittel einzusetzen, wie Nitrobenzol, o-Chlornitrobenzol, 1,2,4-Trichlorbenzol und/oder $CS_2$ (vgl. Houben-Weyl, Bd. V/3, S. 674).

Die Menge des einzusetzenden inerten, organischen Lösungsmittels ist für das erfindungsgemässe Verfahren nicht kritisch und kann in weiten Bereich variiert werden. Es ist lediglich erforderlich, dass eine ausreichende Menge zur Lösung des 1-Alkyl-4-nitrobenzols vorhanden ist. Üblicherweise werden die inerten, organischen Lösungsmittel in einer Menge von etwa 1 bis 10 Liter, bevorzugt 2 bis 4 Liter pro kg einzusetzendes 1-Alkyl-4-nitrobenzol eingesetzt.

Die Chlorierung der 1-Alkyl-4-nitrobenzole kann bei Temperaturen von etwa 0 bis 150 °C, bevorzugt bei 30 bis 80 °C, durchgeführt werden.

Nach dem erfindungsgemässen Verfahren wird die Chlorierung der 1-Alkyl-4-nitrobenzole bis zu einem Gehalt an 1,3,4-Trichlor-2-alkyl- 5-nitrobenzol von mindestens 60 Gew.-%, bevorzugt 70 bis 90 Gew.-%, besonders bevorzugt 75 bis 85 Gew.-% im Chlorierungsgemisch betrieben. Das Molverhältnis von Chlor zu 1-Alkyl-4-nitrobenzol beträgt dabei etwa 2:1 bis 4:1, bevorzugt 2,7:1 bis 3,5:1.

Das bei der Chlorierung anfallende Chlorierungsprodukt wird unter Anwendung üblicher Verfahren wie Waschen mit Wasser (vgl. z.B. Houben-Weyl, Bd. V/3, S. 651–725) von dem eingesetzten Chlorierungskatalysator befreit und anschliessend zur Hydrolyse eingesetzt.

Zur Hydrolyse des erhaltenen Chlorierungsprodukts wird ein Hydrolysegemisch eingesetzt, das aus Wasser und aus einem mit Wasser mischbaren organischen Lösungsmittel besteht sowie eine alkalisch reagierende Verbindung enthält. Als mit Wasser mischbare organische Lösungsmittel können niedere Alkohole, Ketone und/oder cyclische Ether verwendet werden. Bevorzugt werden jedoch $C_1$–$C_5$-Alkohole eingesetzt, wie Methanol, Ethanol, Propanol, Isopropanol, Butanol und/oder Isobutanol, besonders bevorzugt Methanol. Die Menge des bei der Hydrolyse eingesetzten Lösungsmittelgemisches ist nicht kritisch und kann variiert werden. Es ist lediglich erforderlich, dass eine ausreichende Menge eingesetzt wird, um die Rührfähigkeit des Hydrolysegemisches zu gewährleisten.

Als alkalisch reagierende Verbindungen können Alkali- und/oder Erdalkalihydroxide, -oxide, -carbonate und/oder -acetate eingesetzt werden. Bevorzugt werden jedoch die Alkalihydroxide in das erfindungsgemässe Verfahren eingesetzt.

Das Mengenverhältnis der bei der Hydrolyse eingesetzten Reaktionsteilnehmer, d.h. der alkalisch reagierenden Verbindung und des chlorierten Produkts, kann in weiten Bereichen variieren. Die günstige Menge ist leicht durch Vorversuche zu ermitteln. Üblicherweise setzt man etwa 2 bis 10, bevorzugt 5 bis 7, Mol an alkalisch reagierender Verbindung pro Mol hydrolysierbares Chlor ein.

Die Temperatur der Hydrolysereaktion kann ebenfalls in weiten Bereichen schwanken und wird nach unten durch die Reaktionsgeschwindigkeit der Hydrolyse limitiert und nach oben bei drucklosem Arbeiten durch den jeweiligen Siedepunkt des Hydrolysegemisches. Bevorzugt wird die Hydrolyse bei Temperaturen von etwa 20 bis 100, besonders bevorzugt bei 40 bis 80 °C durchgeführt.

Nach Beendigung der Hydrolyse wird das gegebenenfalls bei Kühlung des Hydrolysegemisches ausfallende Reaktionsprodukt abgesaugt, und man erhält in nahezu reiner Form das entsprechende Alkali- oder Erdalkalisalz des 2,4-Dichlor-3-alkyl-6-nitrophenols. Sämtliche aus der Chlorierungsstufe und aus der Hydrolysestufe mit eingeschleppten oder gebildeten Nebenprodukte verbleiben dagegen im Filtrat.

Zur Herstellung der freien Phenole wird das erhaltene Nitrophenolat mit einer wässrigen anorganischen Säure, wie wässriger Salzsäure und/oder wässriger Schwefelsäure, nach bekannten Verfahren behandelt (vgl. z.B. Houben-Weyl, Band VI/1c, S. 146–173).

Das erfindungsgemässe Verfahren kann durch das folgende Formelschema dargestellt werden:

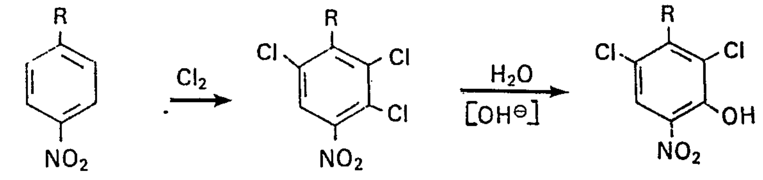

Das erfindungsgemässe Verfahren kann sowohl kontinuierlich als auch diskontinuierlich durchgeführt werden.

Die nach dem erfindungsgemässen Verfahren hergestellten 2,4-Dichlor-3-alkyl- 6-nitrophenole fallen in hoher Reinheit und mit hohen Ausbeuten an.

Im Vergleich zu den bekannten Verfahren (z.B. DE-OS 2 216 804 und DE-OS 2 501 829), die ausgehend von m-Methylphenol das 2,4-Dichlor-3-methyl-6-nitrophenol herstellen, wird bei dem erfindungsgemässen Verfahren die Anzahl der Reaktionsstufen entscheidend verringert, was zur Wirtschaftlichkeit des erfindungsgemässen Verfahrens beiträgt. Ausserdem fehlen beim erfindungsgemässen Verfahren arbeitsaufwendige Trennverfahren, wie Feinvakuumdestillationen. Weiterhin ist die im Rahmen der Erfindung vorgeschlagene Arbeitsweise ohne den Einsatz von Spezialapparaturen, wie sie beispielsweise bei der Isomerisierung von Alkylphenolen zu einem o-, m-, p-Alkylphenol-Isomerengemisch aufgrund der sehr hohen Temperaturen benötigt werden, durchführbar.

Bei dem erfindungsgemässen Verfahren ist es besonders überraschend, dass die Chlorierung von 1-Alkyl-4-nitrobenzol mit Chlor zu einem besonders hohen Gehalt an 1,3,4-Trichlor-2-alkyl-5-nitrobenzolen im Chlorierungsgemisch führt (etwa 75 bis 85% 1,3,4-Trichlor-2-alkyl- 5-nitrobenzol neben 5 bis 10% Dichlor-1-alkyl-4-nitrobenzol und 5 bis 20% Tetrachlor-1-alkyl-4-nitrobenzol). Weiterhin ist es überraschend, dass die Hydrolyse des 1,3,4-Trichlor- 2-alkyl-5-nitrobenzols mit einem $C_2$–$C_8$-Alkylrest selektiv zum 2,4-Dichlor-3-alkyl- 6-nitrophenol führt, da bei der Hydrolyse des 1,3,4-Trichlor- 2-methyl-5-nitrobenzols nicht das entsprechende 2,4-Dichlor-3-methyl- 6-nitrophenol gebildet wird, sondern durch Dehydrierung ein 10-fach substituiertes Diphenyl.

Die nach dem erfindungsgemässen Verfahren erhaltenen, neuen 2,4-Dichlor-3-alkyl- 6-nitrophenole können in bekannter Weise durch Reduktion zu den entsprechenden Aminoverbindungen umgesetzt werden, die als Zwischenprodukte bei der Herstellung von Cyankupplern für Fotopapiere Verwendung finden (vgl. z.B. DE-OS 2 028 601).

Beispielsweise kann die Reduktion der 2,4-Dichlor-3-alkyl- 6-nitrophenole der Formel (I) zu den entsprechenden 2,4-Dichlor-3-alkyl-6-aminophenolen der Formel (III)

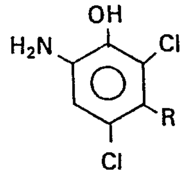

(III),

in der

R für einen Alkylrest mit 2 bis 8 Kohlenstoffatomen steht, katalytisch, z.B. mit einem Palladium/Aktivkohle-Katalysator oder mit einem Raney-Nickel-Katalysator oder durch Eisenreduktion in Eisessig durchgeführt werden.

Die 2,4-Dichlor-3-alkyl- 6-aminophenole bzw. deren Hydrochloride der Formel (III) sind ebenfalls neu. Beispielsweise seien folgende neue Verbindungen genannt: 2,4-Dichlor-3-ethyl- 6-aminophenol, 2,4-Dichlor-3-isopropyl- 6-aminophenol, 2,4-Dichlor-3-tert.- butyl-6-aminophenol, 2,4-Dichlor-3-cyclohexyl- 6-aminophenol und 2,4-Dichlor-3-isoamyl- 6-aminophenol, bevorzugt 2,4-Dichlor-3-ethyl- 6-aminophenol, 2,4-Dichlor-3-isopropyl- 6-aminophenol und 2,4-Dichlor-3-tert.- butyl-6-aminophenol.

Die nachfolgenden Beispiele sollen das erfindungsgemässe Verfahren erläutern, ohne es jedoch auf die Beispiele einzuschränken.

Beispiel 1

906 g (6 Mol) 1-Ethyl-4-nitro-benzol wurden unter Zusatz von 18 g Fe und 0,5 g $J_2$ bei 35 °C mit Chlor umgesetzt, bis das 1,3,4-Trichlor-2-ethyl- 5-nitro-benzol einen Anteil von 82–85% im Rohprodukt ausmachte; hierfür wurden ca. 3,2 Mol Chlor pro Mol Einsatzmaterial benötigt. Das Reaktionsprodukt wurde mit Wasser/HCl oder Wasser neutral und eisenfrei gewaschen. Man erhielt auf diesem Wege 1465 g Rohprodukt folgender Zusammensetzung:

| | |
|---|---|
| 5,8%<br>1,9% | isomere Dichlor-ethyl-nitro-benzole |
| 83,6% | Trichlor-ethyl-nitro-benzol |
| 6,4% | Tetrachlor-ethyl-nitro-benzol. |

Beispiel 2

495 g (3 Mol) 4-Nitro-cumol wurden unter Zusatz von 8 g $SbCl_3$ und 0,5 g $J_2$ bei 38 °C mit elementarem Chlor chloriert, bis das 2,3,6-Trichlor-4-nitro-cumol einen Anteil von 76% im Rohprodukt ausmachte, hierfür wurden ca. 3,65 Mol Chlor pro Mol Einsatzmaterial benötigt. Das Reaktionsprodukt wurde mit konzentriertem HCl oder danach mit Wasser neutral und katalysatorfrei gewaschen. Man erhielt auf diesem Wege 817,3 g Rohprodukt folgender Zusammensetzung:

| | |
|---|---|
| 5,2%<br>0,8% | isomere Dichlor-nitro-cumole |
| 75,3% | 2,3,6-Trichlor-4-nitro-cumol |
| 18,7% | Tetrachlor-nitro-cumol. |

Beispiel 3

Zu einer Lösung von 906 g (6 Mol) 1-Ethyl-4-nitro-benzol in 2000 ml Tetrachlorethan wurden 18 g Fe und 1 g $J_2$ gegeben, danach wurde bei 40 °C mit der Einleitung von Chlor begonnen. Nachdem die Zusammensetzung des Rohproduktes dem von Beispiel 1 entsprach, wurde die Chlorierung beendet und durch Zugabe von Wasser/HCl oder Wasser neutral und eisenfrei gewaschen. Nach Abdestillieren des Lösungsmittels gegebenenfalls im Vakuum erhielt man 1462 g Rohprodukt mit der in Beispiel 1 angegebenen Zusammensetzung.

Beispiel 4

Man legte in einem Rundkolben eine Mischung aus 900 ml Methanol, 100 ml $H_2O$ und 134 g KOH vor, erhitzte auf Rückflusstemperatur (73 °C) und liess sehr schnell unter Rühren 101,8 g Rohprodukt aus Beispiel 1 zulaufen. Nach 4 Stunden Rückfluss war die Lösung dunkelrot geworden, und es hatte sich ein roter Niederschlag, das Kaliumsalz des entstandenen Nitrophenols, ausgeschieden. Man kühlte auf Raumtemperatur ab, saugte das Kaliumnitrophenolat ab, wusch zuerst mit 2 × 50 ml Methanol, dann mit 75 ml kaltem Wasser, überführte anschliessend den wasserfeuchten roten Feststoff in einen zweiten Rundkolben und verrührte ihn dort bei 70–75 °C mit 200 ml 20%iger Schwefelsäure. Nach ca. 3 Stunden hatte sich aus dem Kaliumnitrophenolat das freie Phenol gebildet, das sich als Schmelze (Fp. ca. 45 °C) am Boden des Kolbens ansammelte und durch Schichttrennen abgetrennt werden konnte.

| Elementaranalyse: | ber. | gef. |
|---|---|---|
| C | 40,7% | 40,7% |
| H | 2,9% | 2,9% |
| N | 5,9% | 5,9% |
| Cl | 30,1% | 30,2% |

Schmelzpunkt: F: 45 °C.

Ausbeute: 61,8 g ≙ 79%, bezogen auf die eingesetzte Menge an Trichlor-ethyl-nitro-benzol; Reinheit 97–98%.

Beispiel 5

Man legte in einem Rundkolben eine Mischung aus 900 ml Methanol, 100 ml $H_2O$ und 80 g NaOH vor, erhitzte auf Rückflusstemperatur und liess sehr schnell unter Rühren 103,5 g 1,3,4-Trichlor-2-ethyl-5-nitro-benzol, destillierte Ware aus dem Rohprodukt von Beispiel 1, als Schmelze (38 °C) zufliessen. Nach 4 Stunden Rückfluss war die Lösung dunkelrot gefärbt und kein Einsatzmaterial mehr nachweisbar. Man kühlte auf Raumtemperatur ab, gab zu der Reaktionsmischung 2000 ml $H_2O$, säuerte mit 50%iger Schwefelsäure auf pH 1 an und extrahierte dann mit 3 × 200 ml Chloroform. Nach Eindampfen des Lösungsmittels und anschliessender Wasserdampfdestillation erhielt man 59,4 g 2,4-Dichlor-3-ethyl-6-nitrophenol ≙ 63% der Theorie als 96%ige Ware.

Beispiel 6

Ersetzte man in Beispiel 5 die Menge Methanol durch Ethanol, so betrug die Ausbeute an gewünschtem Endprodukt 53%.

Beispiel 7

Ersetzte man in Beispiel 5 die Menge an NaOH durch eine äquivalente Menge Pottasche oder Natriumacetat, so fand ebenfalls eine Hydrolyse zum 2,4-Dichlor- 3-ethyl- 6-nitrophenol statt; hierbei war jedoch zur Erzielung eines vollständigen Umsatzes eine erheblich längere Reaktionszeit erforderlich.

Beispiel 8

Ersetzte man in Beispiel 5 die Menge Methanol durch das gleiche Volumen Aceton, so erhielt man nach 4-stündiger Reaktionszeit in siedendem Lösungsmittelgemisch eine Ausbeute an 24% 2,4-Dichlor- 3-ethyl- 6-nitro-phenol.

Beispiel 9

Zu einer siedenden Lösung aus 450 ml Methanol, 50 ml $H_2O$ und 67 g KOH wurden unter Rühren schnell 53,7 g Chlorierungsrohprodukt aus Beispiel 2 mit einer Zusammensetzung von 6% Dichlornitro-cumol, 75,3% 2,3,6-Trichlor- 3-nitro-cumol und 18,7% Tetrachlor-nitro-cumol hinzugegeben. Nach 4 Stunden Rückfluss hatte sich eine dunkelrote Suspension gebildet, die dann auf Raumtemperatur abgekühlt und anschliessend abgesaugt wurde. Der abgesaugte Feststoff wurde mit 100 ml methanolischer Kalilauge gewaschen und danach mit 120 ml 20%iger $H_2SO_4$ und 300 ml Chloroform kräftig verrührt. Nach Abtrennen der wässrigen Phase und Eindampfen des Lösungsmittels erhielt man 11,4 g 2,4-Dichlor- 3-isopropyl- 6-nitro-phenol in einer Reinheit von 97–98%; das Filtrat des abgesaugten Feststoffs beinhaltete noch 13,6 g Produkt, so dass insgesamt eine Ausbeute von 65,4% der Theorie erreicht wurde.

| Elementaranalyse: | ber. | gef. |
|---|---|---|
| C | 43,2% | 43,4% |
| H | 3,6% | 3,6% |
| N | 5,5% | 5,2% |
| Cl | 28,4% | 28,4% |

Schmelzpunkt: F: 33 °C.

Beispiel 10

Hydrierung zum 2,4-Dichlor-3-ethyl- 6-aminophenol-hydrochlorid

34,4 g 2,4-Dichlor- 3-ethyl- 6-nitrophenol wurden in 180 ml Methanol unter Zugabe von 3 g Raney-Nickel bei Raumtemperatur und 10 bar $H_2$ im Autoklaven hydriert. Wenn kein Wasserstoff mehr aufgenommen wurde, wurde entspannt, nach Zugabe von 0,4 ml Hydrazinhydrat unter Schutzgas vom Kontakt abfiltriert und dieser mit 20 ml Methanol nachgewaschen. Zum Filtrat gab man unter Kühlen bei 20 °C 200 ml konz. HCl und fällte somit das Amin als Hydrochlorid. Man nutschte wiederum unter Schutzgas ab, wusch das Hydrochlorid mit ca. 200 ml gekühltem Aceton und trocknete im Vakuum.

Ausbeute 27,3 g Amin · HCl, abgenutschte Ware, Reinheit ⩾ 99% (HPLC) ≙ 82% der Theorie.

| Elementaranalyse: | ber. | gef. |
|---|---|---|
| C | 39,6% | 39,4% |
| H | 4,1% | 4,5% |
| N | 5,7% | 5,8% |
| Cl | 43,9% | 43,1% |

Schmelzpunkt: F: 180 °C Zers.

Beispiel 11

2,4-Dichlor- 3-isopropyl- 6-aminophenol-hydrochlorid

80 g (0,31 Mol) 2,4-Dichlor-3-isopropyl-6-nitrophenol wurden mit 425 ml Methanol und 5 g Raney-Nickel bei 25 bis 30 °C und 10 bar $H_2$ im Autoklaven hydriert. Nachdem kein Wasserstoff mehr aufgenommen wurde, rührte man noch 30 Minuten nach, entspannte und filtrierte nach Zugabe von 0,5 ml Hydrazinhydrat zur Hydrierlösung unter Schutzgas vom Kontakt ab, wusch diesen mit 50 ml Methanol nach und säuerte unter Kühlen auf 15 bis 20 °C mit 200 ml konz. HCl an. Die ausfallenden Kristalle wurden abgesaugt, mit 200 ml gekühltem Aceton gewaschen und getrocknet. Man erhielt als Kristallisat I 53,5 g einer 98%igen Ware; dieses sind 70% der Theorie. Durch Eindampfen des Waschacetons erhielt man noch etwa 11–12 g einer 85 bis 90%igen Ware, dies entsprechend 15% der Theorie. Die Hydriermutterlauge wurde verworfen.

| Elementaranalyse: | ber. | gef. |
|---|---|---|
| C | 42,1% | 42,3% |
| H | 4,7% | 4,8% |
| N | 5,4% | 5,4% |
| Cl | 41,5% | 41,4% |

Schmelzpunkt: F: 220 °C Zers.

Beispiel 12

2,4-Dichlor-3-t.-butyl- 6-aminophenol-hydrochlorid

64,8 g (0,24 Mol) 2,4-Dichlor-3-t.-butyl-6-nitrophenol wurden in 400 ml Methanol mit 5 g Raney-Nickel im Autoklaven bei Raumtemperatur und 10 bar $H_2$ hydriert. Nachdem keine Wasserstoffaufnahme mehr stattfand, rührte man noch 30 Minuten nach, entspannte und filtrierte nach Zugabe von 0,5 ml Hydrazinhydrat unter Schutzgas vom Kontakt ab, wusch diesen mit 50 ml Methanol nach und versetzte dann unter Kühlen auf 20 °C die Hydriermutterlauge mit 200 ml konz. Salzsäure. Die ausgefallenen Kristalle wurden abgesaugt, mit 200 ml gekühltem Aceton gewaschen und getrocknet.

Ausbeute: Kristallisat I: 52,2 g als 96%ige Ware ≙ 80% der Theorie.

| Elementaranalyse: | ber. | gef. |
|---|---|---|
| C | 44,4% | 44,8% |
| H | 5,1% | 5,1% |
| N | 5,1% | 5,15% |
| Cl | 39,3% | 39,2% |

Schmelzpunkt: F: 205 °C Zers.

## Patentansprüche

1. Verfahren zur Herstellung von 2,4-Dichlor-3-alkyl- 6-nitrophenolen der Formel

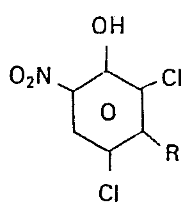 ,

in der

R für einen Alkylrest mit 2 bis 8 Kohlenstoffatomen steht, dadurch gekennzeichnet, dass man 1-Alkyl-4-nitrobenzole der Formel

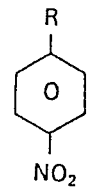 ,

in der

R die obengenannte Bedeutung hat, in Gegenwart eines Katalysators mit Chlor bis zu einem Gehalt an Trichloralkylnitrobenzol von mindestens 60 Gew.-% im Chlorierungsgemisch chloriert, das Chlorierungsgemisch vom Katalysator befreit, anschliessend das Chlorierungsgemisch mit einem Hydrolysegemisch, das aus Wasser und aus einem mit Wasser mischbaren organischen Lösungsmittel sowie einer alkalisch reagierenden Verbindung besteht, hydrolysiert, das ausfallende Reaktionsprodukt abtrennt und mit einer wässrigen anorganischen Säure behandelt.

2. Verfahren nach Anspruch 1, dadurch gekennzeichnet, dass man als Katalysator Eisen(II)chlorid, Antimon(III)chlorid, Aluminium(III)chlorid und/oder Jod einsetzt.

3. Verfahren nach Ansprüchen 1 und 2, dadurch gekennzeichnet, dass man den Katalysator in einer Menge von 1 bis 10 Gew.-%, bezogen auf das eingesetzte 1-Alkyl-4-nitrobenzol, einsetzt.

4. Verfahren nach Ansprüchen 1 bis 3, dadurch gekennzeichnet, dass man die Chlorierung der 1-Alkyl-4-nitrobenzole bis zu einem Gehalt von 70 bis 90 Gew.-% an 1,3,4-Trichlor-2-alkyl-5-nitrobenzol im Chlorierungsgemisch durchführt.

5. Verfahren nach Ansprüchen 1 bis 4, dadurch gekennzeichnet, dass man Chlor zu 1-Alkyl-4-nitrobenzol im Molverhältnis von 2:1 bis 4:1 einsetzt.

6. Verfahren nach Ansprüchen 1 bis 5, dadurch gekennzeichnet, dass man die Chlorierung bei Temperaturen von 0 bis 150 °C durchführt.

7. Verfahren nach Ansprüchen 1 bis 6, dadurch gekennzeichnet, dass man die alkalisch reagierende Verbindung in einer Menge von 2 bis 10 Mol pro Mol hydrolysierbares Chlor einsetzt.

## Revendications

1. Procédé de production de 2,4-dichloro-3-alkyl- 6-nitrophénols de formule

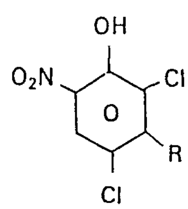 ,

dans laquelle

R représente un reste alkyle de 2 à 8 atomes de carbone, caractérisé en ce qu'on chlore des 1-alkyl-4-nitrobenzènes de formule

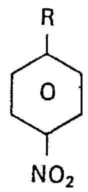
,

dans laquelle

R a la définition mentionnée ci-dessus, en présence d'un catalyseur avec du chlore jusqu'à une teneur en trichloralkylnitrobenzène d'au moins 60% en poids dans le mélange de chloration, on débarrasse le mélange de chloration du catalyseur puis on hydrolyse le mélange de chloration avec un mélange d'hydrolyse qui est constitué d'eau et d'un solvant organique miscible à l'eau ainsi que d'un composé à réaction alcaline, on sépare le produit de réaction qui précipite et on le traite avec une solution aqueuse d'acide inorganique.

2. Procédé suivant la revendication 1, caractérisé en ce qu'on utilise comme catalyseur le chlorure de fer (III), le chlorure d'antimoine (III), le chlorure d'aluminium (III) et/ou l'iode.

3. Procédé suivant les revendications 1 et 2, caractérisé en ce qu'on utilise le catalyseur en une quantité de 1 à 10% en poids par rapport au 1-alkyl-4-nitrobenzène utilisé.

4. Procédé suivant les revendications 1 à 3, caractérisé en ce qu'on effectue la chloration des 1-alkyl-4-nitrobenzènes jusqu'à une teneur en 1,3,4-trichloro-2-alkyl- 5-nitrobenzène de 70 à 90% en poids dans le mélange de chloration.

5. Procédé suivant les revendications 1 à 4, caractérisé en ce qu'on utilise un rapport molaire du chlore au 1-alkyl-4-nitrobenzène de 2:1 à 4:1.

6. Procédé suivant les revendications 1 à 5, caractérisé en ce qu'on effectue la chloration à des températures de 0 à 150 °C.

7. Procédé suivant les revendications 1 à 6, caractérisé en ce qu'on utilise le composé à réaction alcaline en une quantité de 2 à 10 moles par mole de chlore hydrolysable.

## Claims

1. Process for the preparation of 2,4-dichloro-3-alkyl- 6-nitrophenols of the formula

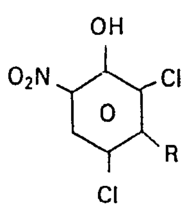

in which

R represents an alkyl radical having 2 to 8 carbon atoms, characterized ... . itrobenzenes of the formula

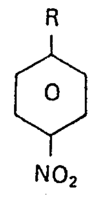

in which

R has the abovementioned meaning, are chlorinated, with chlorine in the presence of a catalyst, up to a content of trichloroalkylnitrobenzene of at least 60% by weight in the chlorination mixture, the catalyst is removed from the chlorination mixture, then the chlorination mixture is hydrolysed with a hydrolysis mixture which consists of water and of an organic solvent shich is miscible with water, as well as of a compound having an alkaline reaction, and the precipitated reaction product is removed and treated with an aqueous inorganic acid.

2. Process according to Claim 1, characterized in that the catalyst used is iron(III) chloride, antimony(III) chloride, aluminium(III) chloride and/or iodine.

3. Process according to Claims 1 and 2, characterized in that the amount of catalyst used is 1 to 10% by weight relative to the 1-alkyl-4-nitrobenzene used.

4. Process according to Claims 1 to 3, characterized in that the chlorination of the 1-alkyl-4-nitrobenzenes is carried out up to a content of 70 to 90% by weight of 1,3,4-trichloro- 2-alkyl- 5-nitrobenzene in the chlorination mixture.

5. Process according to Claims 1 to 4, characterized in that the molar ratio of chlorine to 1-alkyl-4-nitrobenzene used is 2:1 to 4:1.

6. Process according to Claims 1 to 5, characterized in that the chlorination is carried out at temperatures from 0 to 150 °C.

7. Process according to Claims 1 to 6, characterized in that the amount of the compound having an alkaline reaction which is used is 2 to 10 moles per mole of hydrolysable chlorine.